# FASCICULE DE BREVET EUROPEEN

(11) **EP 4 090 218 B1**
(45) Date de publication et mention de la délivrance du brevet: **26.02.2025**
(21) Numéro de dépôt: 20853533.6
(22) Date de dépôt: 22.12.2020
(51) Int. Cl.: A61B 1/00, A61B 1/005, A61B 1/015

(54) **POIGNÉE AVEC MÉCANISME DE COMMANDE DE LA FLEXION DE LA TÊTE D'UN ENDOSCOPE MÉDICAL**
HANDGRIFF MIT EINEM MECHANISMUS ZUR STEUERUNG DER BEUGUNG DES KOPFES EINES MEDIZINISCHEN ENDOSKOPS
HANDLE WITH MECHANISM FOR CONTROLLING THE FLEXION OF THE HEAD OF A MEDICAL ENDOSCOPE

(30) Priorité: 17.01.2020 FR 2000446
(43) Date de publication de la demande: 23.11.2022
(73) Titulaire: Axess Vision Technology, 37300 Joue les Tours (FR)
(72) Inventeur: HALLAUER, Emmanuel, 37190 Sache (FR)
(74) Mandataire: Cabinet Beau de Loménie
(86) Numéro de dépôt international: PCT/FR2020/052593
(87) Numéro de publication internationale: WO 2021/144513

(56) Documents cités:
- WO-A1-2011/089349
- US-A- 5 618 258
- US-A1- 2013 324 973

## Description

### Domaine Technique

La présente invention concerne le domaine technique des endoscopes médicaux au sens général permettant d'accéder à l'intérieur d'un organe creux, d'une cavité ou d'un conduit naturel ou artificiel du corps humain en vue d'effectuer diverses opérations à des fins thérapeutiques, chirurgicales ou de diagnostic.

La présente invention concerne plus précisément la poignée de commande de tels endoscopes, adaptée pour rester à l'extérieur de l'organe creux, de la cavité ou du conduit à inspecter et équipée en particulier d'un mécanisme de commande de la flexion de la tête d'un endoscope médical.

L'endoscope selon l'invention est utilisé à des fins de diagnostic, thérapeutiques ou de chirurgie pour l'inspection de toutes les parties internes du corps humain accessibles par les voies naturelles ou artificielles. Par exemple, l'endoscope selon l'invention peut être utilisé dans le domaine des voies urinaires, des voies gastro-intestinales, du système respiratoire, du système cardiovasculaire, de la trachée, de la cavité du sinus, du système de reproduction de la femme, de la cavité abdominale ou de tout autre partie du corps humain à explorer par une voie naturelle ou artificielle.

### Technique antérieure

D'une manière générale, un endoscope comporte une poignée de commande se présentant sous la forme d'un boîtier adapté pour être tenu par un utilisateur de l'endoscope et auquel est fixée une structure tubulaire conçue pour être insérée dans une cavité corporelle d'un patient à examiner. Cette structure tubulaire d'insertion comporte une tête distale équipée d'un système de vision permettant d'éclairer et d'examiner l'organe, la cavité ou le conduit du corps humain. En amont de la tête distale, la structure tubulaire d'insertion comporte une partie de béquillage commandée par un mécanisme de la poignée de commande pour orienter la tête distale à l'intérieur de la voie d'insertion.

L'état de la technique a proposé de nombreuses solutions pour réaliser le mécanisme permettant de commander la flexion de la tête distale de la structure tubulaire d'insertion. Par exemple, le brevet US 6 017 322 décrit un mécanisme de commande comportant une molette rotative agissant sur une poulie sur laquelle sont fixées les extrémités de deux câbles dont les autres extrémités sont fixées à la tête distale de la structure tubulaire d'insertion.

Un autre exemple de mécanisme simple est décrit dans le brevet EP 1 804 639 comportant une paire de fils de commande reliés à une structure de transmission comprenant une pluralité de corps reliés entre eux par des maillons de manière à coulisser entre eux. Ce mécanisme est commandé par une molette rotative s'étendant à l'extérieur de la poignée.

Dans le document WO 2010/066789, la poignée comporte un levier déplaçable selon un trajet incurvé et relié à un levier pouvant pivoter autour d'un axe de pivotement et agissant sur deux fils de commande pour assurer la flexion de la tête distale.

La demande de brevet US 2013/324973 décrit une poignée pour un cathéter comportant un boîtier formé de deux demi-coques délimitant un logement pour un mécanisme d'actionnement annulaire pour des câbles entraînant la flexion de la tête distale. Le mécanisme d'actionnement annulaire comporte un élément de base et un couvercle assemblés ensemble et délimitant intérieurement un canal de retenue et de glissement pour les parties terminales des câbles. La face intérieure d'une demi-coque est pourvue d'un fut tubulaire autour duquel tourne le mécanisme d'actionnement. Ce mécanisme d'actionnement est pourvu de deux boutons diamétralement opposés, faisant saillie du boîtier pour être accessibles par l'opérateur. Un système de tension prend appui sur une demi-coque tandis qu'une vis est engagée à travers l'autre demi-coque et dans le fut tubulaire pour coopérer avec le système de tension. Le serrage du système de tension permet de régler la force de compression appliquée par les demi-coques sur le mécanisme d'actionnement annulaire et, par suite d'ajuster la capacité de rotation de ce mécanisme d'actionnement

La demande brevet WO 2011/089349 décrit un endoscope comportant un système pour actionner la tête distale du tube d'insertion comportant des poulies montées coaxiales dans le boîtier de la poignée de commande. Ces poulies sont entraînées en rotation par des leviers rotatifs de commande s'étendant latéralement et sur l'arrière de la poignée.

Quel que soit le mode de réalisation du mécanisme de commande, il doit être considéré que l'actionnement du bouton de commande est une opération délicate à mener à bien par l'opérateur compte tenu du fait que l'opérateur doit tenir fermement la poignée de commande pendant l'opération d'inspection. Dans de nombreuses solutions, l'utilisateur a une difficulté pour exercer sur le bouton de commande, un effort adapté pour assurer la flexion de la tête distale tout en maintenant la poignée. Une difficulté supplémentaire apparaît lorsque l'utilisateur doit par ailleurs agir sur le dispositif d'obturation d'un circuit de circulation de fluide pour amener des fluides ou pour aspirer des fluides au niveau de la tête distale.

### Exposé de l'invention

La présente invention vise donc à remédier aux inconvénients de l'état de la technique en proposant une nouvelle poignée de commande pour un endoscope médical, conçue pour faciliter les opérations de commande visant à assurer la flexion de la tête distale.

Pour atteindre un tel objectif, la poignée pour endoscope médical comporte les caractéristiques de la revendication 1, à savoir un boîtier présentant deux faces principales opposées et pourvu d'un mécanisme de commande de la flexion d'une tête distale de l'endoscope médical, ce mécanisme de commande comportant au moins un organe d'actionnement d'une pièce pivotante réalisée sous la forme d'une bague d'actionnement présentant une virole axiale présentant une surface externe cylindrique coopérant avec un palier annulaire pour être guidée en rotation par ce palier annulaire, selon un axe transversal de rotation et dont la rotation entraîne la flexion de la tête distale, la virole axiale présentant un alésage intérieur délimitant en partie une ouverture cylindrique configurée pour l'insertion d'un doigt et qui traverse de part en part le boîtier en débouchant par des alésages intérieurs sur les deux faces principales opposées du boîtier, la pièce pivotante et le palier annulaire étant aménagés pour être situés à l'extérieur de l'ouverture cylindrique.

Selon une caractéristique de réalisation, l'ouverture cylindrique possède une section de passage comprise entre 8 x 8 mm et 30 x 30 mm et de préférence entre 8 x 8 mm et 25 x 25 mm.

Typiquement, la pièce pivotante est guidée en déplacement par une liaison pivot autour d'un axe transversal de rotation confondu avec l'axe transversal de l'ouverture cylindrique, la pièce pivotante étant bloquée en translation d'un côté, par le palier annulaire monté solidaire d'une première demi-coque du boîtier et de l'autre côté, par une deuxième demi-coque du boîtier.

Avantageusement, la virole axiale de la bague d'actionnement présente à son extrémité libre, un bord d'appui coopérant avec un bord de butée d'un col du palier annulaire pour former ensemble un cylindre vide délimitant intérieurement l'ouverture cylindrique .

Selon un exemple de réalisation, l'organe d'actionnement fait partie d'un collier annulaire fixé sur la pièce pivotante et présentant un alésage intérieur de section identique à l'alésage intérieur de la virole axiale pour former avec la virole axiale et le col du palier, un cylindre vide délimitant intérieurement l'ouverture cylindrique

Par exemple, la première demi-coque et la deuxième demi-coque du boîtier sont fixées ensemble.

Par ailleurs, la pièce pivotante est bloquée transversalement selon un sens, par un bord de butée du palier annulaire aménagé ou fixé sur la première demi-coque et selon l'autre sens, en étant en butée sur la deuxième demi-coque du boîtier.

Selon une variante de réalisation, l'organe d'actionnement est un levier monté solidaire de la pièce pivotante guidée en rotation de manière à déplacer en rotation la pièce pivotante par un mouvement de rotation du levier.

Avantageusement, le levier comporte un bras d'actionnement s'étendant parallèlement à l'axe transversal de rotation et extérieurement au boîtier, entre les deux faces principales du boîtier.

De préférence, l'organe d'actionnement fait partie d'un collier annulaire délimité par un alésage monté solidaire de la pièce pivotante en étant en butée sur la deuxième demi-coque du boîtier.

Selon une autre variante de réalisation, l'organe d'actionnement est guidé en déplacement linéaire et agit sur la pièce pivotante à l'aide d'un système de transformation du mouvement linéaire de l'organe de commande en un mouvement de rotation de la pièce pivotante.

De manière classique, au moins un câble de commande pour la flexion de la tête distale est montée à l'intérieur de la poignée de commande, la pièce pivotante agissant directement ou par un système de transformation de mouvement, sur au moins ledit câble de commande pour la flexion de la tête distale.

Selon une caractéristique avantageuse de réalisation, le boîtier comporte un premier logement pour un circuit de circulation de fluide et un deuxième logement pour un câble électrique, une partie de ces logements passant de part et d'autre de l'ouverture cylindrique, ces logements débouchant, à une partie proximale du boîtier.

Un autre objet de l'invention est de proposer un endoscope médical comportant une structure tubulaire d'insertion supportée par une poignée conforme à l'invention.

Diverses autres caractéristiques ressortent de la description faite ci-dessous en référence aux dessins annexés qui montrent, à titre d'exemples non limitatifs, des formes de réalisation de l'objet de l'invention.

### Brève description des dessins

[Fig. 1]La Figure 1 est une vue en perspective d'un endoscope médical conforme à l'invention.
[Fig. 2]La Figure 2 est une vue en perspective d'un exemple de réalisation d'une poignée de commande conforme à l'invention dont une partie du boîtier est enlevée.
[Fig. 3]La Figure 3 est une vue en perspective de la poignée de commande illustrée à la Fig. 2 dont une partie est enlevée pour montrer une caractéristique de l'objet de l'invention.
[Fig. 4]La Figure 4 est une vue en coupe transversale montrant des détails caractéristiques de la poignée conforme à l'invention.
[Fig. 5]La Figure 5 est une vue en perspective éclatée illustrant la poignée de commande conforme à l'invention.
[Fig. 6]La Figure 6 est un schéma montrant un autre exemple de réalisation du mécanisme de commande de la flexion de la tête distale de l'endoscope.
[Fig. 7]La Figure 7 est un schéma montrant un autre exemple de réalisation du mécanisme de commande de la flexion de la tête distale de l'endoscope, à l'aide d'un organe d'actionnement linéaire.

### Description des modes de réalisation

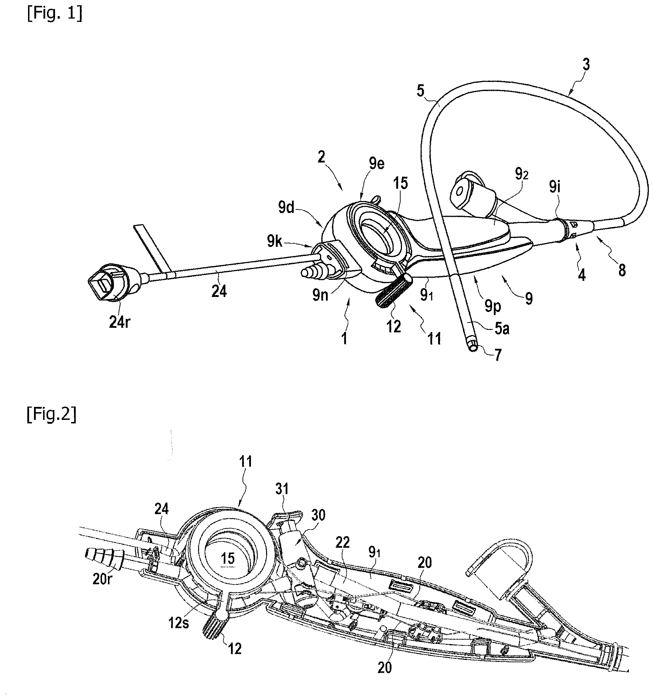

Tel que cela ressort plus précisément des Fig. 1 à 5, l'objet de l'invention concerne un endoscope médical 1 comportant un support d'actionnement 2 tel qu'une poignée de commande équipée d'un instrument médical consommable 3. Cet instrument médical consommable 3 qui est en contact avec les tissus ou les organes humains relèvent essentiellement d'un usage unique ou multiple d'un patient voire d'un usage réutilisable après décontamination, désinfection et voire une stérilisation.

L'instrument médical consommable 3 est assemblé à la poignée 2 de façon définitive ou de façon temporaire à l'aide d'un système d'assemblage 4. Par exemple, le système d'assemblage 4 assure une liaison complète entre la poignée 2 et l'instrument médical consommable 3 ou est du type à encliquetage pour assurer rapidement une liaison au moins mécanique temporaire tout en offrant l'avantage de permettre une séparation facile de l'instrument médical consommable 3 par rapport à la poignée 2.

De manière classique, l'instrument médical consommable 3 comporte un tube d'insertion externe 5 présentant une longueur et une flexibilité plus ou moins importante et destiné à être introduit dans une voie d'accès naturel ou artificiel en vue d'effectuer diverses opérations ou fonctions à des fins thérapeutiques, chirurgicales ou de diagnostic. Par exemple, le tube d'insertion 5 possède une partie distale 7 formant la tête de l'endoscope 1. Le tube d'insertion 5 comporte également une partie proximale 8 opposée à la partie distale 7 et s'étendant en saillie à partir de l'extrémité distale 9i d'un boîtier 9 formant le corps principal de la poignée 2.

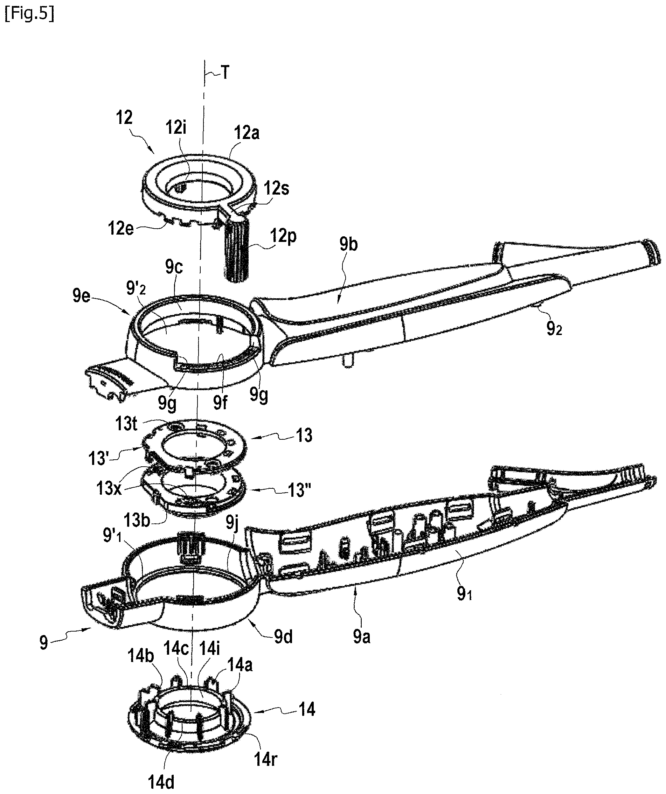

Ce boîtier 9 se présente sous la forme d'un corps allongé aménagé pour présenter une préhension aisée de l'endoscope. Dans l'exemple de réalisation illustré, le boîtier 9 comporte une première demi-coque 9₁ et une deuxième demi-coque 9₂ de forme complémentaire à la première demi-coque 9₁ de manière à pouvoir être assemblées ou fixées ensemble par tous systèmes de fixation tels que par encliquetage par exemple (Fig. 5). La première demi-coque 9₁ et la deuxième demi-coque 9₂ possèdent des sections droites en forme de C. Ce boîtier 9 présente ainsi d'un côté d'un plan longitudinal médian P, une première face principale 9a et du côté opposé de ce plan, une deuxième face principale 9b. Ces deux faces principales opposées 9a, 9b se raccordent par une plage périphérique 9p.

La poignée 2 comporte un mécanisme 11 de commande de la flexion de la tête distale 7 du tube d'insertion 5 permettant d'orienter la tête distale 7 par rapport à l'axe longitudinal du tube d'insertion 5. A cet effet, le tube d'insertion 5 comporte en amont de la tête distale 7, une partie de flexion, de pliage ou de béquillage 5a permettant l'orientation de la tête distale 7 par rapport à l'axe longitudinal du tube d'insertion 5. Cette partie de flexion, de pliage ou de béquillage 5a peut être réalisée de toute manière appropriée pour assurer la flexion de la tête distale 7 par rapport à l'axe longitudinal du tube d'insertion 5. Par exemple, cette partie de flexion, de pliage ou de béquillage 6 peut être réalisée par un ressort ou par des vertèbres tubulaires articulées entre elles.

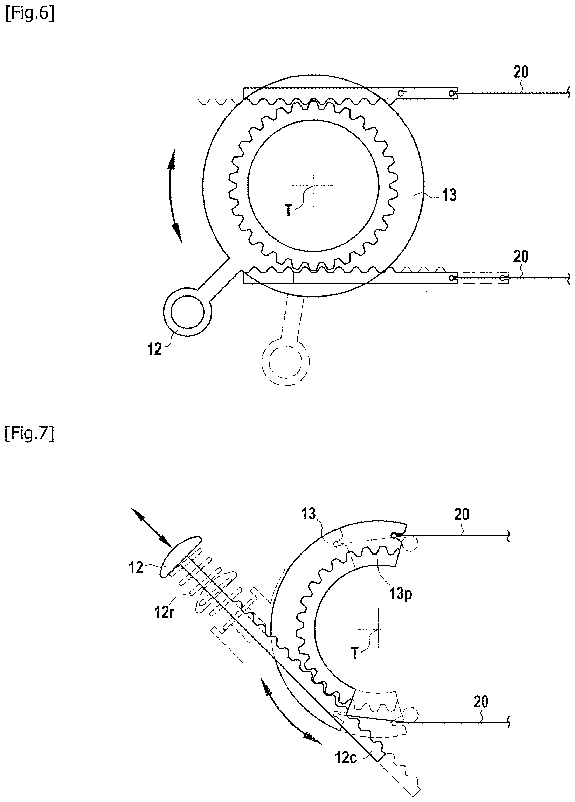

Conformément à l'invention, le mécanisme de commande 11 comporte au moins un organe d'actionnement 12 qui à la suite de l'application d'un effort manuel, entraîne la flexion de la tête distale 7. Selon l'exemple de réalisation illustré aux Fig. 1 à 5, l'organe d'actionnement 12 est un levier guidé en rotation sur une plage angulaire limitée, selon un axe transversal de rotation T. Selon l'exemple illustré à la Fig. 7, l'organe d'actionnement 12 est un poussoir guidé en déplacement linéaire et sollicité en rappel élastique.

L'organe d'actionnement 12 agit sur une pièce pivotante 13 de manière à entraîner directement ou indirectement, sa rotation autour de l'axe transversal de rotation T. Cette pièce pivotante 13 est reliée à la tête distale 7 de sorte qu'une rotation de la pièce pivotante 13 entraîne la flexion de la tête distale 7. Cette pièce pivotante 13 est guidée en rotation par un palier de forme annulaire 14.

Selon une caractéristique de l'invention, la pièce pivotante 13 et le palier annulaire 14 sont aménagés pour être situés à l'extérieur d'une ouverture ou d'un trou cylindrique 15 qui traverse de part en part le boîtier 9 pour déboucher sur les deux faces principales opposées 9a, 9b du boîtier. Tel que cela ressort clairement des Figures, l'ouverture cylindrique 15 traverse complètement le boîtier 9 en s'ouvrant sur les deux faces principales opposées 9a, 9b du boîtier et correspondant à un espace vide. L'ouverture cylindrique 15 traverse ainsi le palier annulaire 14, la pièce pivotante 13, la première demi-coque 9₁ et la deuxième demi-coque 9₂. Comme il sera expliqué dans la suite de la description, l'opérateur peut ainsi y insérer un doigt pour faciliter les opérations de préhension et/ou de manœuvre de l'endoscope. Typiquement, l'ouverture cylindrique 15 possède une section circulaire avec un diamètre compris entre 10 et 40 mm et de préférence entre 10mm et 25mm pour permettre notamment l'insertion d'un doigt d'un opérateur ou d'un crochet pour suspendre l'endoscope médical 1. Dans le cas où l'ouverture cylindrique 15 n'est pas de section circulaire, l'ouverture cylindrique 15 possède une section de passage comprise entre 8mmx8mm et 30mmx30mm et de préférence entre 8mmx8mm et 25mmx25mm.

L'ouverture cylindrique 15 comporte un axe transversal qui est avantageusement confondu avec l'axe transversal de rotation T de la pièce pivotante 13. Dans l'exemple illustré, l'ouverture cylindrique 15 possède une section circulaire mais il est clair que la section de l'ouverture cylindrique 15 peut être de forme différente.

Selon un exemple de réalisation illustré sur les Fig. 1 à 5, le levier 12 entraîne directement en rotation la pièce pivotante 13 autour de l'axe transversal de rotation T. Selon cet exemple, le levier 12 fait partie d'un collier annulaire 12a délimité par un alésage intérieur 12ᵢ, destiné à être fixé par tous moyens appropriés sur la pièce pivotante 13. Par exemple, le collier annulaire 12a comporte des ergots 12b adaptés à s'engager dans des trous 13t aménagés dans la pièce pivotante 13. Bien entendu, le levier 12 peut faire partie intégrante de la pièce pivotante 13, comme dans la variante de réalisation illustrée à la Fig. 6 dans laquelle la pièce pivotante 13 est une bague pourvue radialement d'un bras formant un levier 12 d'actionnement en rotation.

Selon un autre exemple de réalisation illustré à la Fig. 7, l'organe d'actionnement 12 peut entraîner indirectement la rotation de la pièce pivotante 13 autour de l'axe transversal de rotation T. Selon cet exemple, l'organe d'actionnement 12 qui est un poussoir déplaçable en translation et rappelé en position par un ressort 12r, agit sur la pièce pivotante 13 par l'intermédiaire d'un système de transformation du mouvement linéaire du poussoir en un mouvement de rotation de la pièce pivotante 13. Ce système de transformation comporte un pignon 13p et une crémaillère 12c fixés respectivement à la pièce pivotante 13 et à l'organe d'actionnement 12. Il doit être considéré que par exemple l'organe d'actionnement 12 fait partie d'un collier annulaire 12a monté autour de la pièce pivotante 13 et délimité par un alésage intérieur 12i délimitant en partie l'ouverture cylindrique 15 qui traverse de part en part le boîtier.

Selon une caractéristique avantageuse de réalisation, la pièce pivotante 13 est guidée par une liaison pivot de manière à présenter uniquement un mouvement de rotation autour de l'axe transversal de rotation T. La pièce pivotante 13 peut être réalisée de différentes manières en considérant que le mouvement de rotation de la pièce pivotante est limité généralement à une amplitude angulaire inférieure à un tour et en particulier, inférieure à un tiers d'un tour. Selon l'exemple de réalisation illustré aux Fig. 1 à 5, la pièce pivotante 13 est réalisée sous la forme d'une bague comportant un anneau complet ou fermé 13a. Bien entendu, la pièce pivotante 13 peut être réalisée de manière différente comme sous la forme d'une semi-bague ou d'une portion d'anneau notamment, comme illustré selon la variante de réalisation de la Fig. 7 par exemple.

Cette bague 13 est guidée en rotation par une virole axiale 13b coopérant avec le palier annulaire 14. Cette virole axiale 13b s'étend en saillie axialement par rapport à l'anneau 13a de la bague en étant centré selon l'axe transversal de rotation T. La virole axiale 13b présente une surface extérieure cylindrique 13e coopérant avec le palier annulaire 14 pour assurer le guidage en rotation de la bague.

A cet effet, le palier annulaire 14 présente un guidage annulaire formé par une série de nervures 14a axiales présentant chacune un bord intérieur axial 14b. Ces bords intérieurs radiaux 14b sont distribués sur un cercle centré selon l'axe transversal de rotation T afin de coopérer avec la surface extérieure cylindrique 13e de la virole axiale.

L'anneau 13a de la bague 13 est délimité à sa périphérie par un bord extérieur 13₁ et dans sa partie centrale, par un alésage intérieur 13₂. Il est à noter que l'alésage intérieur 13₂ délimite la surface intérieure de la virole axiale 13b, qui est coaxiale à la surface externe cylindrique 13e. Il s'ensuit que la bague 13 comporte un alésage intérieur 13₂ formant une partie de l'ouverture cylindrique 15 qui traverse de part en part le boîtier. La pièce pivotante 13 est ainsi guidée extérieurement par l'intérieur du palier annulaire 14 permettant de délimiter un alésage intérieur 13₂ avec un diamètre important et correspondant à un espace vide.

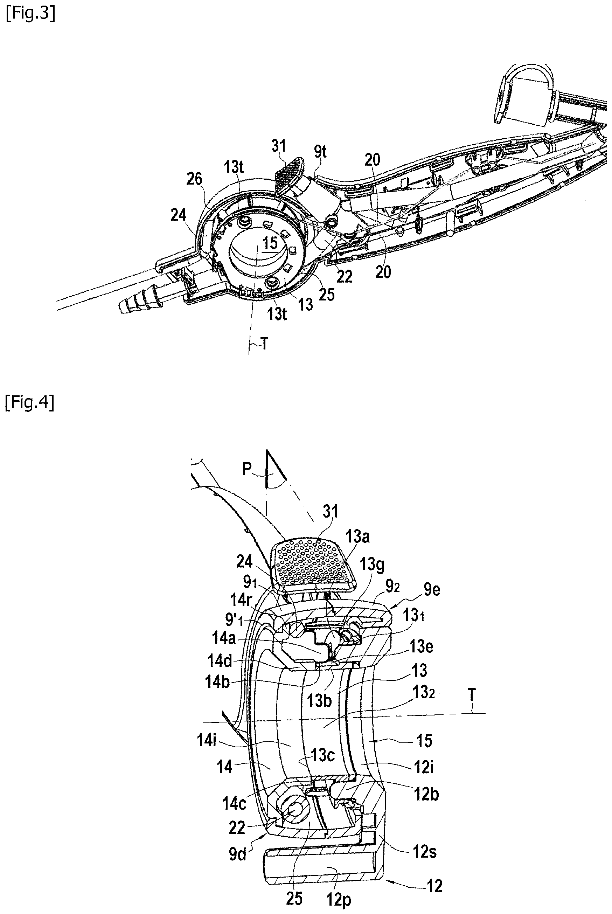

Selon une caractéristique avantageuse de réalisation, la virole axiale 13b de la bague 13 présente à son extrémité libre, un bord d'appui 13c coopérant avec un bord de butée 14c du palier annulaire 14. Tel que cela ressort plus précisément de la Fig. 4, le palier annulaire 14 comporte ainsi un col cylindrique 14d s'étendant axialement en étant centré selon l'axe transversal de rotation T et se terminant à son extrémité libre, par le bord de butée 14c. Il est à noter que les nervures 4a sont portées par la surface extérieure cylindrique du col 14d. Ce col 14d présente un alésage intérieur 14i. Ainsi, le palier annulaire 14 délimite ainsi par son alésage intérieur 14i, une partie de l'ouverture cylindrique 15 qui traverse de part en part le boîtier 9.

Avantageusement, la bague 13 présente un alésage intérieur 13₂ de section identique à l'alésage intérieur 14₁ du palier annulaire 14. Il s'ensuit que la bague 13 et le palier annulaire 14 qui sont juxtaposés en venant dans le prolongement l'un de l'autre, délimitent ensemble l'ouverture cylindrique 15 qui débouche dans la première demi-coque 9₁ et dans la deuxième demi-coque 9₂. Ainsi, la virole axiale 13b de la bague d'actionnement coopère par son bord d'appui 13c, avec le bord de butée 14c du col cylindrique 14d du palier annulaire pour former ensemble un cylindre vide délimitant intérieurement l'ouverture cylindrique 15.

Dans l'exemple de réalisation illustré, le levier 12 est rapporté par un collier annulaire 12a fixé sur la bague 13. Comme cela apparaît à la Fig. 4, l'alésage intérieur 12i du collier annulaire 12a possède une section identique à l'alésage intérieur 13₂ de la bague 13 de sorte que la bague 13, le palier annulaire 14 et le collier annulaire 12a délimitent ensemble l'ouverture cylindrique 15 qui traverse de part en part le boîtier. Le collier annulaire 12a vient par sa partie intérieure délimitant l'alésage intérieur 12i, en appui ou dans le prolongement de la virole axiale 13b, du côté opposé au bord d'appui 13c. Il s'ensuit que le collier annulaire 12a délimite par l'alésage intérieur 12i avec la virole axiale 13b de la bague 13 et le col 14d du palier, un cylindre vide délimitant intérieurement l'ouverture cylindrique 15.

Selon l'exemple de réalisation illustré sur les dessins, le palier annulaire 14 est fixé sur une face principale du boîtier et plus précisément à la première demi-coque 9₁ du boîtier. A cet effet, le palier annulaire 14 se présente sous la forme d'un enjoliveur annulaire rapporté et fixé par tous moyens appropriés sur une demi-coque du boîtier par exemple la première demi-coque 9₁. Par exemple, la première demi-coque 9₁ présente un trou de montage 9'₁ pour l'enjoliveur 14 qui comporte par exemple, à sa périphérie, des ergots radiaux 14r bloqués axialement par des pattes 9j aménagées sur la face interne de la première demi-coque 9₁ à la périphérie du trou de montage 9'₁. Par exemple, la première demi-coque 9₁ est aménagée pour présenter une anse ou un collier circulaire 9d formant une saillie ou un bossage et s'étendant axialement en étant centré sur l'axe transversal de rotation T et délimitant le trou de montage 9'₁ de section circulaire. Le palier annulaire 14 est donc monté à l'intérieur du trou de montage 9'₁ de la première demi-coque 9₁ et délimité par l'anse ou le collier circulaire 9d.

Selon un exemple de réalisation non illustré sur les dessins, le palier annulaire 14 peut être aménagé directement sur la première demi-coque 9₁. Selon cet exemple, la première demi-coque présente sur sa face interne toutes les caractéristiques du palier annulaire 14 décrites ci-dessus.

II est à noter que la bague 13 est bloquée en translation, par le bord de butée 14c du palier annulaire 14 qui est solidaire de la première demi-coque 9₁, selon un sens de l'axe transversal de rotation T, à savoir celui dirigé vers la première demi-coque 9₁ dans l'exemple considéré. La bague 13 est également bloquée en translation selon l'autre sens de l'axe transversal de rotation T, à savoir celui dirigé vers la deuxième demi-coque 9₂ dans l'exemple considéré. Ainsi, la bague 13 est en butée sur la deuxième face principale 9b du boîtier tout en conservant sa liberté de rotation par rapport au boîtier.

Dans l'exemple de réalisation illustré, la bague 13 est en butée sur la deuxième face principale du boîtier et plus précisément sur la deuxième demi-coque 9₂, par l'intermédiaire du collier annulaire 12a de l'organe d'actionnement, fixé à la bague 13. Selon cet exemple, le collier annulaire 12a comporte des ergots 12e répartis à sa périphérie pour venir en appui contre un rebord 9c d'un trou 9'₂ aménagé dans la deuxième demi-coque 9₂. Par exemple, la deuxième demi-coque 9₂ est aménagée pour présenter une anse ou un collier circulaire 9e formant une saillie ou un bossage et s'étendant axialement en étant centré sur l'axe transversal de rotation T et délimitant le trou de montage 9'₂ de section circulaire. Le collier annulaire 12a est donc monté à l'intérieur du trou de montage 9'₂ aménagé dans la deuxième demi-coque 9₂ et délimité par l'anse ou le collier circulaire 9e.

II ressort des dessins que l'anse ou le collier circulaire 9d aménagé dans la première demi-coque 9₁ et l'anse ou le collier circulaire 9e aménagé dans la deuxième demi-coque 9₂ forment ensemble un fût transversal tubulaire 9d, 9e traversé par l'ouverture cylindrique 15. Le boîtier 9 présente ainsi à sa partie proximale, le fût transversal 9d, 9e qui se prolonge en direction de l'extrémité distale 9d du boîtier 9, par un corps allongé. Le fût transversal 9d, 9e du boîtier 9 est prolongé en direction opposée de l'extrémité distale 9i, par un nez proximal 9n délimitant l'extrémité proximale 9k du boîtier.

Selon une variante avantageuse de réalisation, l'organe d'actionnement 12 et en particulier le bras d'appui 12p présente une surface antidérapante, réalisée par exemple par le matériau constitutif du bras d'appui 12p (en caoutchouc par exemple) ou par une surface texturée (par un moletage par exemple). Cette surface antidérapante garantit une bonne accroche avec le pouce lors de la phase de béquillage pendant l'examen malgré les gants du praticien ou les fluides pouvant rendre le bras d'appui glissant.

Selon une autre caractéristique avantageuse de réalisation, le levier d'actionnement 12 comporte un bras d'appui 12p s'étendant parallèlement à l'axe transversal de rotation T et extérieurement au boîtier, entre les deux faces principales du boîtier. A cet effet, le collier annulaire 12a de l'organe d'actionnement est pourvu d'un segment radial 12s à l'extrémité duquel s'étend perpendiculairement le bras d'appui 12p qui se trouve positionné à proximité de la plage périphérique 9p du boîtier. Comme cela apparaît sur les dessins, le bras d'appui 12p qui possède un déplacement selon un arc de cercle, est apte à se déplacer en regard d'une partie des anses ou colliers circulaires 9e, 9d du boîtier.

Avantageusement, l'anse ou le collier circulaire 9e de la deuxième demi-coque 9₂ est pourvue d'une échancrure 9f délimitée de part et d'autre par des épaulements 9g et dans laquelle prend place le segment radial 12s du levier d'actionnement 12. Les épaulements 9g constituent ainsi des butées de fin de course pour le levier d'actionnement 12. Cette échancrure 9f est aménagée sur une portion limitée de l'anse ou du collier circulaire 9e, par exemple de l'ordre de 45°, en étant située dans la partie inférieure du boîtier lorsque la poignée est en position d'utilisation.

La pièce pivotante 13 agit directement ou par un mécanisme de transformation, sur au moins un et dans l'exemple illustré deux câbles 20 de commande pour assurer la flexion de la tête distale 7. Dans l'exemple de réalisation illustré aux Fig. 2 à 5, les extrémités des câbles 20 sont fixées à la pièce pivotante 13 de toute manière appropriée de sorte qu'une rotation de la pièce pivotante 13 entraîne la flexion de la tête distale 7. Avantageusement, chaque câble 20 passe dans une gorge 13g aménagée dans le bord extérieur 13₁ de la bague 13. La pièce pivotante 13 forme ainsi une poulie annulaire pivotante assurant par exemple le déplacement gauche-droit ou bas-haut de la tête 7. Bien entendu, les câbles 20 sont réalisés de toute manière appropriée pour assurer cette fonction de flexion. Ainsi ces câbles 20 peuvent être montés dans une gaine et être réalisés par des tiges, fils ou chaînes, réalisés en un matériau métallique ou polymère par exemple.

Les câbles d'actionnement 20 sont montés à l'intérieur de la poignée de commande 2 en y sortant par l'extrémité distale 9i pour pénétrer à l'intérieur du tube d'insertion 5 et être fixés par leurs extrémités distales à la partie distale 7 du tube d'insertion. Les extrémités proximales des câbles d'actionnement 20 sont fixées à la pièce pivotante 13 de toute manière appropriée. Dans l'exemple illustré, la pièce pivotante 13 est réalisée par deux pièces assemblées ensemble à savoir un flasque annulaire mâle 13' pourvu de tétons d'assemblage et un flasque annulaire femelle 13" pourvu de logements de réception des tétons du flasque annulaire mâle. Au moins l'un des flasques annulaires 13', 13" est aménagé pour comporter deux logements 13x adaptés pour recevoir chacun un manchon serti sur l'extrémité d'un câble 20. Ces logements 13x qui sont fermés lors de l'assemblage des deux flasques annulaires 13', 13" assurent l'ancrage des câbles 20 à la pièce pivotante 13.

Il ressort de la description qui précède que la poignée conforme à l'invention présente une ergonomie de manipulation optimisée grâce à l'ouverture cylindrique 15 qui correspond à un espace vide traversant de part en part le boîtier de la poignée. Cette ouverture cylindrique 15 autorise en particulier une bonne préhension de la poignée tout en offrant une facilité pour la manœuvre de l'organe d'actionnement 12 qui est situé à proximité de l'ouverture cylindrique 15. Cette ouverture cylindrique 15 offre également une possibilité d'accrochage de la poignée sur un crochet de tous types connus en soi.

Il doit être noté également que cette poignée 2 présente une symétrie longitudinale permettant une préhension ambidextre. De même, l'actionnement de l'organe d'actionnement 12 peut être effectué aussi facilement de la main gauche que de la main droite.

Par ailleurs, cette poignée 1 offre une bonne résistance à l'écrasement par la coopération avec le fût transversal tubulaire 9d, 9e des demi-coques, de la pièce pivotante 13 et du palier annulaire 14. Toutefois, il est à noter que cette poignée présente un poids réduit compte tenu de la présence de l'ouverture cylindrique 15 permettant de réduire la masse du matériau constitutif des demi-coques du boîtier.

Par ailleurs, il est à noter que le levier d'actionnement 12 est monté facilement sur la pièce pivotante 13 qui se trouve guidée en rotation sur une plage angulaire importante permettant d'obtenir une précision pour la rotation de la pièce pivotante 13.

Dans la description qui précède, la poignée 1 comporte un mécanisme de commande 11 pour béquiller la tête distale 7 selon une direction mais il est clair que la poignée peut comporter deux mécanismes de commande 11 pour béquiller la tête distale 7 selon deux directions perpendiculaires.

Selon une autre caractéristique de l'invention, la poignée 1 comporte un circuit 22 de circulation de fluide adapté pour amener du fluide jusqu'à la tête distale 7 ou aspirer des fluides à partir de la tête distale 7. Ce circuit de circulation de fluide 22 est monté à l'intérieur du boîtier, entre l'extrémité proximale 9k et l'extrémité distale 9ᵢ pour se prolonger à l'intérieur du tube d'insertion 5 jusqu'à la tête distale 7. De même, la poignée 2 est apte à permettre d'éclairer et de ramener une image de la partie distale 7 du tube d'insertion 5. A cet effet, un câble électrique 24 est monté à l'intérieur du boîtier, entre l'extrémité proximale et l'extrémité distale pour se prolonger à l'intérieur du tube d'insertion 5 jusqu'à la tête distale 7.

Selon une autre caractéristique de l'invention, le boîtier 9 est aménagé pour permettre le positionnement du circuit de circulation de fluide 22 d'un côté de l'ouverture cylindrique 15 et le positionnement du câble électrique 24, de l'autre côté de l'ouverture cylindrique 15. A cet effet, le boîtier 9 et en en particulier, la première demi-coque 9₁ au niveau de l'anse ou du collier circulaire 9e, est aménagée de manière à délimiter d'un côté du trou de montage 9'₁, un premier logement 25 pour le passage du circuit de circulation de fluide 20 et de l'autre côté du trou de montage 9'₁, un deuxième logement 26 pour le câble électrique 24. Ces logements 25, 26 qui possèdent chacun la forme d'une demi-couronne se rejoignent dans la partie allongée du boîtier dans laquelle le circuit de circulation de fluide 20 et le câble électrique 24 sont montés. Le circuit de circulation de fluide 20 et le câble électrique 24 traversent l'extrémité distale 9i du boîtier pour être insérés dans le tube d'insertion 5. Ces logements 25, 26 se rejoignent également du côté opposé, pour déboucher dans le nez proximal 9n du boîtier et permettre le montage à l'extérieur du boîtier, d'un raccord 24r sur le câble électrique 24 et d'un raccord 20r sur le circuit de circulation de fluide 20 pour son raccordement à une source d'aspiration ou d'amenée d'un fluide.

Il ressort de la description qui précède un bon équilibrage des masses à l'intérieur de la poignée 2 dans la mesure où notamment les sorties du circuit de circulation de fluide 20 et du câble électrique 24 sont situées à l'extrémité proximale 9k du boîtier, c'est-à-dire à l'opposé de l'extrémité distale 9i du boîtier à travers laquelle sortent le circuit de circulation de fluide 20 et le câble électrique 24. Le circuit de circulation de fluide 20 et le câble électrique 24 sont ainsi montés à l'intérieur du boîtier 9 d'une extrémité 9k à l'autre extrémité 9i, en passant de part et d'autre de l'ouverture cylindrique 15. Le positionnement de ces sorties à l'extrémité proximale 9k du boîtier offre une bonne qualité de préhension de la poignée.

De manière classique, le circuit de circulation de fluide 20 est pourvu d'un obturateur 30 contrôlant l'ouverture et la fermeture du circuit 20 à l'aide d'un bouton de commande 31 déplaçable en translation et faisant saillie du boîtier. Avantageusement, le boîtier 9 est aménagé pour présenter un orifice 9t bordant le fût transversal tubulaire 9d, 9e des demi-coques de manière que le bouton de commande 31 s'étende de manière tangente à ce fût transversal tubulaire 9d, 9e des demi-coques, dans la partie supérieure du boîtier lorsque la poignée est en position d'utilisation. Ainsi, ce bouton de commande 31 peut être facilement actionné par la main déplaçant également le levier 12 pour la flexion de la tête distale 7.

L'invention n'est pas limitée aux exemples décrits et représentés, l'invention est limitée uniquement par les revendications annexées.

## Revendications

1. Poignée pour endoscope médical comportant un boîtier (9) présentant deux faces principales opposées (9ₐ, 9_{b}) et pourvu d'un mécanisme de commande (11) de la flexion d'une tête distale (7) de l'endoscope médical, ce mécanisme de commande (11) comportant au moins un organe d'actionnement (12) d'une pièce pivotante (13) réalisée sous la forme d'une bague d'actionnement (13a) présentant une virole axiale (13b) présentant une surface externe cylindrique (13e) coopérant avec un palier annulaire (14) pour être guidée en rotation par ce palier annulaire (14), selon un axe transversal de rotation (T) et dont la rotation entraîne la flexion de la tête distale, la virole axiale (13b) présentant un alésage intérieur (13₂) délimitant en partie une ouverture cylindrique (15) qui traverse de part en part le boîtier (9) en débouchant par des alésages intérieurs (12i, 14i) sur les deux faces principales opposées (9₁, 9₂) du boîtier, la pièce pivotante (13) et le palier annulaire (14) étant aménagés pour être situés à l'extérieur de l'ouverture cylindrique (15).

2. Poignée selon la revendication 1, selon laquelle l'ouverture cylindrique (15) possède une section de passage comprise entre 8 x 8 mm et 30 x 30 mm et de préférence entre 8 x 8 mm et 25 x 25 mm.

3. Poignée selon l'une des revendications précédentes, selon laquelle la pièce pivotante (13) est guidée par une liaison pivot autour d'un axe transversal de rotation (T) confondu avec l'axe transversal de l'ouverture cylindrique (15), la pièce pivotante (13) étant bloqué en translation d'un côté, par le palier annulaire (14) monté solidaire d'une première demi-coque (9₁) du boîtier et de l'autre côté, par une deuxième demi-coque (9₂) du boîtier.

4. Poignée selon la revendication précédente, selon laquelle la virole axiale (13b) de la bague d'actionnement présente à son extrémité libre, un bord d'appui (13c) coopérant avec un bord de butée (14c) d'un col (14d) du palier annulaire pour former ensemble un cylindre vide délimitant intérieurement l'ouverture cylindrique (15).

5. Poignée selon la revendication précédente, selon laquelle l'organe d'actionnement (12) fait partie d'un collier annulaire (12a) fixé sur la pièce pivotante (13) et présentant un alésage intérieur (12i) de section identique à l'alésage intérieur (13₂) de la virole axiale (13b) pour former avec la virole axiale (13b) et le col (14d) du palier, un cylindre vide délimitant intérieurement l'ouverture cylindrique (15).

6. Poignée selon l'une des revendications précédentes, selon laquelle la première demi-coque (9₁) et la deuxième demi-coque (9₂) du boîtier sont fixées ensemble.

7. Poignée selon la revendication précédente, selon laquelle la pièce pivotante (13) est bloquée transversalement selon un sens, par un bord de butée (14c) du palier annulaire (14) aménagé ou fixé sur la première demi-coque (9₁) et selon l'autre sens, en étant en butée sur la deuxième demi-coque (9₂) du boîtier.

8. Poignée selon l'une des revendications précédentes, selon laquelle l'organe d'actionnement (12) est un levier monté solidaire de la pièce pivotante (13) guidée en rotation de manière à déplacer en rotation la pièce pivotante (13) par un mouvement de rotation du levier.

9. Poignée selon la revendication précédente, selon laquelle le levier (12) comporte un bras d'actionnement (12p) s'étendant parallèlement à l'axe transversal de rotation (T) et extérieurement au boîtier, entre les deux faces principales du boîtier.

10. Poignée selon l'une des revendications précédentes, selon laquelle l'organe d'actionnement (12) fait partie d'un collier annulaire (12a monté solidaire de la pièce pivotante (13) en étant en butée sur la deuxième demi-coque (9₂) du boîtier.

11. Poignée selon l'une des revendications 1 à 8, selon laquelle l'organe d'actionnement (12) est guidé en déplacement linéaire et agit sur la pièce pivotante à l'aide d'un système de transformation (12c, 13p) du mouvement linéaire de l'organe de commande en un mouvement de rotation de la pièce pivotante.

12. Poignée selon l'une des revendications précédentes, selon laquelle au moins un câble (20) de commande pour la flexion de la tête distale est monté à l'intérieur de la poignée de commande, la pièce pivotante (13) agissant directement ou par un système de transformation de mouvement, sur au moins ledit câble (20) de commande pour la flexion de la tête distale.

13. Poignée selon l'une des revendications précédentes, selon laquelle le boîtier comporte un premier logement (25) pour un circuit de circulation de fluide (20) et un deuxième logement (26) pour un câble électrique (24), une partie de ces logements passant de part et d'autre de l'ouverture cylindrique (15), ces logements (25, 26) débouchant, à une partie proximale (9n) du boîtier.

14. Endoscope médical comportant une poignée (2) selon l'une des revendications précédentes et une structure tubulaire d'insertion (3) supportée par ladite poignée (2).

## Patentansprüche

1. Griff für medizinisches Endoskop, umfassend ein Gehäuse (9), das zwei gegenüberliegende Hauptflächen (9ₐ, 9_{b}) aufweist und das mit einem Steuermechanismus (11) der Biegung eines distalen Kopfs (7) des medizinischen Endoskops versehen ist, dieser Steuermechanismus (11) umfassend mindestens ein Betätigungselement (12) eines schwenkbaren Teils (13) umfasst, das in Form eines Betätigungsrings (13a) ausgeführt ist, der einen axialen Ring (13b) aufweist, der eine zylindrische Außenfläche (13e) aufweist, die mit einem ringförmigen Lager (14) zusammenwirkt, um durch dieses ringförmige Lager (14) in Drehung um eine transversale Drehachse (T) geführt zu werden, und dessen Drehung die Biegung des distalen Kopfs bewirkt, wobei der axiale Ring (13b) eine innere Bohrung (13₂) aufweist, die teilweise eine zylindrische Öffnung (15) begrenzt, die das Gehäuse (9) durchquert, indem sie durch innere Bohrungen (12i, 14i) an den zwei gegenüberliegenden Hauptflächen (9₁, 9₂) des Gehäuses mündet, wobei das schwenkbare Teil (13) und das ringförmige Lager (14) angeordnet sind, um sich außerhalb der zylindrischen Öffnung (15) zu befinden.

2. Griff nach Anspruch 1, wobei die zylindrische Öffnung (15) einen Durchgangsquerschnitt zwischen 8 x 8 mm und 30 x 30 mm und vorzugsweise zwischen 8 x 8 mm und 25 x 25 mm aufweist.

3. Griff nach einem der vorherigen Ansprüche, wobei das schwenkbare Teil (13) durch eine Schwenkverbindung um eine transversale Drehachse (T) geführt wird, die mit der transversalen Achse der zylindrischen Öffnung (15) zusammenfällt, wobei das schwenkbare Teil (13) auf der einen Seite durch das ringförmige Lager (14), das fest mit einer ersten Halbschale (9₁) des Gehäuses verbunden ist, und auf der anderen Seite durch eine zweite Halbschale (9₂) des Gehäuses in Translation blockiert ist.

4. Griff nach dem vorherigen Anspruch, wobei der axiale Ring (13b) des Betätigungsrings an seinem freien Ende einen Stützrand (13c) aufweist, der mit einem Anschlagrand (14c) eines Halses (14d) des ringförmigen Lagers zusammenwirkt, um gemeinsam einen leeren Zylinder zu bilden, der die zylindrische Öffnung (15) innen begrenzt.

5. Griff nach dem vorherigen Anspruch, wobei das Betätigungselement (12) Teil eines ringförmigen Bunds (12a) ist, der an dem schwenkbaren Teil (13) befestigt ist, und eine Innenbohrung (12i) mit gleichem Querschnitt wie die Innenbohrung (13₂) des axialen Rings (13b) aufweist, um mit dem axialen Ring (13b) und dem Hals (14d) des Lagers einen leeren Zylinder zu bilden, der die zylindrische Öffnung (15) innen begrenzt.

6. Griff nach einem der vorherigen Ansprüche, wobei die erste Halbschale (9₁) und die zweite Halbschale (9₂) des Gehäuses aneinander befestigt sind.

7. Griff nach dem vorherigen Anspruch, wobei das schwenkbare Teil (13) quer in einer Richtung durch einen Anschlagrand (14c) des ringförmigen Lagers (14), der an der ersten Halbschale (9₁) angeordnet oder befestigt ist, und in der anderen Richtung durch Anliegen an der zweiten Halbschale (9₂) des Gehäuses blockiert ist.

8. Griff nach einem der vorherigen Ansprüche, wobei das Betätigungselement (12) ein Hebel ist, der fest mit dem drehbar geführten schwenkbaren Teil (13) montiert ist, um das schwenkbare Teil (13) durch eine Drehbewegung des Hebels in Drehung zu versetzen.

9. Griff nach dem vorherigen Anspruch, wobei der Hebel (12) einen Betätigungsarm (12p) umfasst, der sich parallel zu der transversalen Drehachse (T) und außerhalb des Gehäuses zwischen den zwei Hauptflächen des Gehäuses erstreckt.

10. Griff nach einem der vorherigen Ansprüche, wobei das Betätigungselement (12) Teil eines ringförmigen Bunds (12a) ist, der fest mit dem schwenkbaren Teil (13) montiert ist, indem er an der zweiten Halbschale (9₂) des Gehäuses anliegt.

11. Griff nach einem der Ansprüche 1 bis 8, wobei das Betätigungselement (12) linear beweglich geführt wird und mittels eines Umwandlungssystems (12c, 13p) der linearen Bewegung des Betätigungselements in eine Drehbewegung des schwenkbaren Teils auf das schwenkbare Teil einwirkt.

12. Griff nach einem der vorherigen Ansprüche, wobei mindestens ein Steuerkabel (20) für die Biegung des distalen Kopfs im Inneren des Steuergriffs montiert ist, das schwenkbare Teil (13) direkt oder über ein Bewegungsumwandlungssystem auf mindestens das Steuerkabel (20) für die Biegung des distalen Kopfs einwirkt.

13. Griff nach einem der vorherigen Ansprüche, wobei das Gehäuse eine erste Aufnahme (25) für einen Fluidkreislauf (20) und eine zweite Aufnahme (26) für ein Stromkabel (24) umfasst, ein Teil dieser Aufnahmen auf beiden Seiten der zylindrischen Öffnung (15) verläuft, wobei diese Aufnahmen (25, 26) an einem proximalen Teil (9n) des Gehäuses münden.

14. Medizinisches Endoskop, umfassend einen Griff (2) nach einem der vorherigen Ansprüche und eine röhrenförmigen Einführstruktur (3), die von dem Griff (2) getragen wird.

## Claims

1. A handle for a medical endoscope including a casing (9) having two opposite main faces (9ₐ, 9_{b}) and provided with a mechanism for controlling (11) the bending of a distal head (7) of the medical endoscope, this control mechanism (11) including at least one member for actuating (12) a pivoting part (13) made in the form of an actuation ring (13a) having an axial ferrule (13b) having a cylindrical outer surface (13e) cooperating with an annular bearing (14) to be guided in rotation by this annular bearing (14), along a transverse axis of rotation (T) and whose rotation causes the bending of the distal head, the axial ferrule (13b) having an internal bore (13₂) partly delimiting a cylindrical opening (15) which passes right through the casing (9) by opening out through internal bores (12i, 14i) on the two opposite main faces (9₁, 9₂) of the casing, the pivoting part (13) and the annular bearing (14) being arranged to be located outside the cylindrical opening (15).

2. The handle according to claim 1, according to which the cylindrical opening (15) has a passage section comprised between 8 x 8 mm and 30 x 30 mm and preferably between 8 x 8 mm and 25 x 25 mm.

3. The handle according to any of the preceding claims, according to which the pivoting part (13) is guided by a pivot connection about a transverse axis of rotation (T) coincident with the transverse axis of the cylindrical opening (15), the pivoting part (13) being blocked in translation on one side, by the annular bearing (14) mounted secured to a first half-shell (9₁) of the casing and on the other side, by a second half- shell (9₂) of the casing.

4. The handle according to the preceding claim, according to which the axial ferrule (13b) of the actuation ring has at its free end, a bearing edge (13c) cooperating with an abutment edge (14c) of a neck (14d) of the annular bearing to form together an empty cylinder internally delimiting the cylindrical opening (15).

5. The handle according to the preceding claim, according to which the actuation member (12) forms part of an annular collar (12a) fixed on the pivoting part (13) and having an internal bore (12i) of section identical to the internal bore (13₂) of the axial ferrule (13b) to form, with the axial ferrule (13b) and the neck (14d) of the bearing, an empty cylinder internally delimiting the cylindrical opening (15).

6. The handle according to any of the preceding claims, according to which the first half-shell (9₁) and the second half-shell (9₂) of the casing are fixed together.

7. The handle according to the preceding claim, according to which the pivoting part (13) is blocked transversely in one direction by an abutment edge (14c) of the annular bearing (14) arranged or fixed on the first half-shell (9₁) and in the other direction by abutting on the second half-shell (9₂) of the casing.

8. The handle according to any of the preceding claims, according to which the actuation member (12) is a lever mounted secured to the pivoting part (13) guided in rotation so as to move in rotation the pivoting part (13) by a rotational movement of the lever.

9. The handle according to the preceding claim, according to which the lever (12) includes an actuation arm (12p) extending parallel to the transverse axis of rotation (T) and externally to the casing, between the two main faces of the casing.

10. The handle according to any of the preceding claims, according to which the actuation member (12) forms part of an annular collar (12a) mounted secured to the pivoting part (13) by abutting on the second half-shell (9₂) of the casing.

11. The handle according to any of claims 1 to 8, according to which the actuation member (12) is guided in linear displacement and acts on the pivoting part by means of a system for transforming (12c, 13p) the linear movement of the control member into a rotational movement of the pivoting part.

12. The handle according to any of the preceding claims, according to which at least one control cable (20) for the bending of the distal head is mounted inside the control handle, the pivoting part (13) acting directly or by a movement transformation system, on at least said control cable (20) for the bending of the distal head.

13. The handle according to any of the preceding claims, according to which the casing includes a first housing (25) for a fluid circulation circuit (20) and a second housing (26) for an electric cable (24), part of these housings passing on either side of the cylindrical opening (15), these housings (25, 26) opening out at a proximal part (9n) of the casing.

14. A medical endoscope including a handle (2) according to any of the preceding claims and a tubular insertion structure (3) supported by said handle (2).
